# EUROPÄISCHE PATENTSCHRIFT

(11) **EP 0 625 142 B1**
(45) Veröffentlichungstag und Bekanntmachung des Hinweises auf die Patenterteilung: **24.04.1996**
(21) Anmeldenummer: 93903900.4
(22) Anmeldetag: 03.02.1993
(51) Int. Cl.: C07D 201/08, C07D 315/00

(54) **VERFAHREN ZUR HERSTELLUNG VON PYRROLIDON UND N-ALKYLPYRROLIDONEN**
PROCESS FOR PRODUCING PYRROLIDONE AND N-ALKYL PYRROLIDONES
PROCEDE DE FABRICATION DE PYRROLIDONE ET DE N-ALKYLPYRROLIDONES

(30) Priorität: 07.02.1992 DE 4203527
(43) Veröffentlichungstag der Anmeldung: 23.11.1994
(73) Patentinhaber: Akzo Nobel N.V., NL-6824 BM Arnhem (NL)
(72) Erfinder: BERGFELD, Manfred, D-8765 Erlenbach-Mechenhard (DE); WIESGICKL, Günter, D-8751 Grosswallstadt (DE)
(74) Vertreter: Fett, Günter
(86) Internationale Anmeldenummer: EP9300239
(87) Internationale Veröffentlichungsnummer: WO9316042

(56) Entgegenhaltungen:
- EP-A- 0 184 055
- WO-A-86/07358
- DE-A- 2 159 859
- DE-A- 2 200 600
- FR-A- 1 576 183
- GB-A- 931 685
- GB-A- 1 109 540
- US-A- 2 267 757
- US-A- 3 109 005
- US-A- 3 115 500
- US-A- 3 136 780
- US-A- 3 198 808
- US-A- 3 235 562
- US-A- 3 448 118
- DATABASE WPIL Week 9112, Derwent Publications Ltd., London, GB; AN 91-085402
- BULLETIN OF THE CHEMICAL SOCIETY OF JAPAN. Bd. 50, Nr. 10, Oktober 1977, TOKYO JP 'Synthetic zeolites as Catalysts for the Ring Conversion of gamma- Butyrolactone into 1-substituted 2-Pyrrolidones.'
- J. CATALYSIS Bd. 37, 1975, Seiten 166 - 175 HATADA ET. AL. 'Ring Transformation of gamma-Butyrolactone into 2-Pyrrolidone over Zeolites.'
- CHEMISTRY LETTERS 1974, Seiten 439 - 442 HATADA ET. AL. 'Ring Transformations of Oxygen containing heterocycles into nitrogen containing heterocycles over synthetic zeolites.'

## Beschreibung

Die Anmeldung betrifft ein Verfahren zur Herstellung von Pyrrolidon und N-Alkylpyrrolidonen aus gesättigten oder ungesättigten 1.4-Dicarbonsäuren und ihren Anhydriden, Wasserstoff und einem primären Amin oder Ammoniak.

N-Alkylpyrrolidone, insbesondere das N-Methylpyrrolidon sind technisch sehr wichtige Lösungsmittel in der industriellen, erdölverarbeitenden Chemie und sie werden auch als Ausgangsmaterial für verschiedene Synthesen verwendet.

Industriell wird etwa N-Methylpyrrolidon im allgemeinen nach dem Reppe-Verfahren (Chem. Ing. Technik (22) 17, 1950, S. 361 ff) hergestellt, bei dem Monomethylamin und Gamma-Butyrolacton mit einem geeigneten Verdünnungsmittel über einen wasserabspaltenden Katalysator für einen bestimmten Zeitraum auf Temperaturen über 250°C gehalten werden. Durch die reaktionsbedingt leicht erfolgende Dimerisierung des N-Methylpyrrolidons beträgt die Ausbeute jedoch lediglich 85-90%.

In der US 4,885,371 ist ein Verfahren zur Herstellung von N-Methylpyrrolidon beschrieben, bei dem Monomethylamin und Gamma-Butyrolacton über einen Borhydridsalzkatalysator miteinander umgesetzt werden.

Eine Reihe weiterer Druckschriften (JP 01190667, JP 01186864, JP 01186863) beschreibt ebenfalls die Synthese von N-Alkylpyrrolidonen, ausgehend von Gamma-Butyrolacton, mit primären, sekundären oder tertiären Aminen. Die mittlere Reaktionszeit beträgt 3 h bei 250°C.

Nach der sowjetischen Anmeldung Nr. 1558903 erhält man durch Reaktion von Gamma-Butyrolacton und Monomethylamin im Überschuß bei 250°C über einen Y-Zeolithen als Katalysator als Produkt N-Methylpyrrolidon. Nach K. Hatada et al. (Bull. Chem. Soc. Japan 50(10), 1977, S. 2517-2521) wird für die gleiche Reaktion ein Kupfer-ausgetauschter Y-Zeolith als Katalysator verwendet.

Bei dem Verfahren der JP 49020585 erhält man durch Reaktion von mit viel Wasser verdünntem Monomethylamin und Gamma-Butyrolacton bei 250°C und 2 Stunden Reaktionsdauer N-Methylpyrrolidon.

Das Verfahren in der JP 51042107 verwendet Gamma-Butyrolacton, Methylamin und Wasser im Verhältnis von 1:1,4:4, um bei einer Temperatur von 250°C und einem Druck von 45-50kg/cm zum Endprodukt N-Methylpyrrolidon zu gelangen. Der Überschuß an Wasser wird als Träger für die Wiederverwendung des nicht-reagierten Methylamins benutzt.

Gemäß der JP 49000259 erhält man N-Methylpyrrolidon durch Erhitzen eines Gemisches von Gamma-Butyrolacton, Alkylamin und Wasserstoff für 3 Stunden auf 270°C. Als Katalysator wird ein Gemisch aus Kupfer und einem Metalloxid (Cu plus SiO₂, Al₂O₃, SiO₂-Al₂O₃, TiO₂, ZrO₂ oder Cr₂O₃) verwendet. Die Reaktion findet in der Gasphase statt, die Ausbeute beträgt lediglich etwa 60%.

Nach der JP 47021420 wird N-Methylpyrrolidon in 99%iger Ausbeute erhalten, wenn man Monomethylamin und Gamma-Butyrolacton genügend lange in wäßriger Phase miteinander reagieren läßt.

Die JP 49020582 beschreibt ein Verfahren, bei dem man zuerst Monomethylamin mit Gamma-Butyrolacton reagieren läßt, und anschließend eine Cyclisierung, etwa über Aluminiumoxid als Katalysator, erfolgt.

Schließlich beschreibt die DE 2200600 ein Verfahren zur Herstellung von N-Methylpyrrolidon aus Maleinsäureanhydrid, Wasserstoff und einer Aminverbindung, bei der ein Palladium-Kohlenstoff-Katalysator verwendet wird. Bei einer Reaktionstemperatur von 275°C und einem Druck von 119 atm wird eine Ausbeute von lediglich maximal 78,4 % erhalten. Die Reaktion findet in einem Überschuß von Wasser statt.

Zudem sind oft sehr lange Reaktionszeiten vorgesehen und die Reaktion erfolgt bei einem sehr hohen Überdruck, so daß aufwendige verfahrenstechnische Maßnahmen zu ergreifen sind.

Alle vorgenannten Verfahren haben entweder den Nachteil einer geringen Ausbeute oder das Produkt hat eine nur unbefriedigende Reinheit und muß aufwendig nachgereinigt werden.

In ähnlicher Weise wie in der DE-A-22 00 600 beschrieben, werden auch in US-A-3,109,005, US-A-3,448,118 und US-A-3,198,808 Verfahren offenbart, bei denen die Herstellung von Pyrrolidon oder Pyrrolidonen in einem sogenannten Eintopfverfahren durchgeführt wird. Diese Verfahren haben die bereits vorstehend erwähnten Nachteile. Meistens geht jedoch die Synthese aus von Gamma-Butyrolacton, welches zuvor aus 1,4-Butandiol, Maleinsäureanhydrid oder anderen Edukten hergestellt und isoliert werden muß.

Der Erfindung liegt daher die Aufgabe zugrunde, ein Verfahren zur Verfügung zu stellen, mit dem sich N-Alkylpyrrolidone bzw. Pyrrolidon selbst direkt aus den entsprechenden Dicarbonsäuren bzw. ihren Anhydriden ohne die sonst übliche Isolierung eines Zwischenprodukts herstellen lassen und bei dem mit einer hohen Ausbeute an Produkt auch zugleich die hohen Reinheitsforderungen an das Produkt erfüllt werden.

Diese Aufgabe wird erfindungsgemäß dadurch gelöst, daß man in einem geeignetem Reaktor über ein erstes Katalysatorbett die Dicarbonsäuren bzw. ihre Anhydride mit Wasserstoff reagieren läßt und anschließend das nicht-isolierte Zwischenprodukt direkt über ein zweites, nachgeschaltetes Katalysatorbett mit einem primären Amin oder Ammoniak umsetzt.

Mit diesem erfindungsgemäßen Verfahren wird zunächst in bis zu über 98%iger Ausbeute und Reinheit hinter bzw. über dem ersten Katalysatorbett das entsprechende Zwischenprodukt erhalten, welches anschließend im Gasphasenzustand mit dem primären Amin oder Ammoniak über dem zweiten, nachgeschalteten Katalysatorbett zu N-Alkylpyrrolidon oder Pyrrolidon umgesetzt wird. Auch bei diesem zweiten Schritt betragen die Reinheit und die Ausbeute im Mittel jeweils bis zu über 98%.

Es hat sich für das Verfahren als vorteilhaft herausgestellt, wenn für das erste Katalysatorbett ein kupferhaltiger Katalysator verwendet wird.

Ebenfalls hat es sich für das Verfahren als vorteilhaft herausgestellt, wenn für das zweite, nachgeschaltete Katalysatorbett ein siliciumhaltiger Katalysator oder ein Aluminium-Phosphat-Katalysator verwendet wird.

Insbesondere sind Katalysatoren bevorzugt, die eine zeolithische Struktur aufweisen. Die besten Ergebnisse werden jedoch erhalten, wenn für das erste Katalysatorbett ein Kupfer-Chromit-Katalysator und für das zweite Katalysatorbett ein kationenausgetauschter Zeolith vom Typ X oder Y verwendet wird. Mit dieser Kombination ist ein bis zu über 98%iger Umsatz der Reaktanden möglich.

Bevorzugt findet die Reaktion in der Gasphase statt, um eine optimale Umsetzung der Reaktionsteilnehmer zu erreichen.

Um die Reaktion besser kontrollieren zu können, und um eine genügend genaue Dosierung der Reaktionsteilnehmer durchführen zu können, können Verdünnungsmittel, wie etwa Gase, Wasser oder aprotische Lösungsmittel anwesend sein. So hat es sich als Vorteil erwiesen, wenn die Reaktionsteilnehmer des ersten Schrittes, primär jedoch der Wasserstoff, mit Stickstoff verdünnt, in das Reaktionssystem eingeführt werden. Ebenso ist das primäre Amin in verdünnter wäßriger Lösung leichter zu dosieren. Insgesamt wird durch die Anwendung der obengenannten Verdünnungsmittel eine kontinuierlicher ablaufende Reaktion erreicht.

Die Temperatur innerhalb des Reaktors beträgt über dem ersten Katalysatorbett 225 bis 350°C und über dem zweiten Katalysatorbett 225 bis 400°C, wobei für das erste Katalysatorbett 250 bis 300°C und für das zweite Katalysatorbett 260 bis 340°C bevorzugt sind. Hierbei werden die maximalen Raum-Zeit-Ausbeuten erzielt.

Das molare Verhältnis von 1.4-Dicarbonsäure bzw. ihrem Anhydrid zu Wasserstoff beträgt 1 zu 20 bis 1 zu 250, während das molare Verhältnis von Wasserstoff zum primären Amin bzw. Ammoniak 15 zu 1 bis 250 zu 1 beträgt.

Die Reaktion ist weiterhin durch kurze bis sehr kurze Verweilzeiten des jeweiligen Reaktionsgemisches über dem jeweiligen, zugehörigen Katalysatorbett gekennzeichnet. Diese Zeiten betragen jeweils 0,01 bis 15 Sekunden und können beim Einsatz geeigneter Strömungsgeschwindigkeiten und Reaktandenverhältnisse auf Zeiten von unterhalb 3 Sekunden herabgesenkt werden.

Die Reaktion läßt sich auch dadurch vorteilhaft ausführen, indem der Reaktionsdruck lediglich im Bereich von 0,1 bis 10 bar liegt und in den meisten Fällen der allgemeine Raumdruck nicht überschritten wird, so daß sich die Reaktion in einfachen Vorrichtungen oder für geringe Überdrücke geeignete Reaktoren ausführen läßt.

Das erfindungsgemäß hergestellte Produkt läßt sich mit einer Ausbeute und Reinheit von jeweils bis zu über 98% herstellen und enthält als Nebenprodukte lediglich etwas Gamma-Butyrolacton und eventuell Propionsäure, von denen es sich leicht reinigen läßt. Insbesondere bei der Herstellung von N-Methylpyrrolidon aus Maleinsäureanhydrid, Methylamin und Wasser wird das nicht umgesetzte Gamma-Butyrolacton leicht abgetrennt, da es sich während der Destillation zu Gammahydroxy-N-methyl-butylamid umsetzt, das sich mit evtl. noch anderen Nebenprodukten im Destillationssumpf absetzt und zurückbleibt.

Die vorliegende Erfindung wird durch das nachstehende Beispiel näher erläutert.

### Beispiel 1

Ein handelsüblicher Glasreaktor wurde an geeigneter Stelle so mit einer Zufuhröffnung versehen, daß diese zwischen dem ersten und zweiten Katalysatorbett lag.
Als Katalysator für das erste Katalysatorbett wurden 21,95g Cu-Chromit-Katalysator (Hersteller: Alfa) und für das zweite Katalysatorbett 15,9g NaX-Zeolith eingesetzt.
Über das erste Katalysatorbett wurden 1,584 mol/h Wasserstoff und 0,016 mol/h Maleinsäureanhydrid geleitet und durch die Zufuhröffnung zusätzlich 0,017 mol/h Monomethylamin und 0,096 mol/h Wasser.
Die Temperatur über dem ersten Katalysatorbett betrug 275°C und über dem zweiten Katalysatorbett 275°C.
Nach einer gesamten Reaktionszeit von 4 Stunden wurden 6,09 g N-Methylpyrrolidon erhalten. Die Ausbeute betrug 96%.

## Patentansprüche

1. Verfahren zur Herstellung von Pyrrolidon und N-Alkylpyrrolidonen aus gesättigten oder ungesättigten 1.4-Dicarbonsäuren und/oder ihren Anhydriden, Wasserstoff und einem primären Amin oder Ammoniak, dadurch gekennzeichnet, daß man in einem geeigneten Reaktor über ein erstes Katalysatorbett die Dicarbonsäure bzw. ihr Anhydrid mit Wasserstoff reagieren läßt und anschließend das nicht-isolierte Zwischenprodukt direkt über ein zweites, nachgeschaltetes Katalysatorbett mit dem primären Amin oder Ammoniak umsetzt.

2. Verfahren nach Anspruch 1, dadurch gekennzeichnet, daß für das erste Katalysatorbett ein kupferhaltiger Katalysator verwendet wird.

3. Verfahren nach Anspruch 1, dadurch gekennzeichnet, daß für das zweite Katalysatorbett ein siliciumhaltiger Katalysator oder ein Aluminium-Phosphat-Katalysator verwendet wird.

4. Verfahren nach Anspruch 3, dadurch gekennzeichnet, daß der Katalysator eine zeolithische Struktur aufweist.

5. Verfahren nach einem oder mehreren der Ansprüche 1 bis 4, dadurch gekennzeichnet, daß für das erste Katalysatorbett ein Kupfer-Chromit-Katalysator und für das zweite Katalysatorbett ein kationenausgetauschter Zeolith vom Typ X oder Y verwendet wird.

6. Verfahren nach einem oder mehreren der Ansprüche 1 bis 5, dadurch gekennzeichnet, daß die Reaktion in der Gasphase stattfindet.

7. Verfahren nach einem oder mehreren der Ansprüche 1 bis 6, dadurch gekennzeichnet, daß Verdünnungsmittel anwesend sind.

8. Verfahren nach Anspruch 7, dadurch gekennzeichnet, daß es sich bei dem Verdünnungsmittel um Gase, Wasser oder aprotische Lösungsmittel handelt.

9. Verfahren nach einem oder mehreren der Ansprüche 1 bis 8, dadurch gekennzeichnet, daß die Temperatur innerhalb des Reaktors über dem ersten Reaktorbett 225 bis 350°C beträgt.

10. Verfahren nach einem oder mehreren der Ansprüche 1 bis 9, dadurch gekennzeichnet, daß die Temperatur innerhalb des Reaktors über dem zweiten Katalysatorbett 225 bis 400°C beträgt.

11. Verfahren nach einem oder mehreren der Ansprüche 1 bis 10, dadurch gekennzeichnet, daß die Temperatur über dem ersten Katalysatorbett 250 bis 300°C und über dem zweiten Katalysatorbett 260 bis 340°C beträgt.

12. Verfahren nach einem oder mehreren der Ansprüche 1 bis 11, dadurch gekennzeichnet, daß das molare Verhältnis der 1.4-Dicarbonsäure bzw. ihrem Anhydrid zu Wasserstoff von 1 zu 20 bis 1 zu 250 beträgt.

13. Verfahren nach einem oder mehreren der Ansprüche 1 bis 12, dadurch gekennzeichnet, daß das molare Verhältnis von Wasserstoff zum primären Amin bzw. Ammoniak 15 zu 1 bis 250 zu 1 beträgt.

14. Verfahren nach einem oder mehreren der Ansprüche 1 bis 13, dadurch gekennzeichnet, daß die Verweilzeit der Reaktionsteilnehmer über dem jeweiligen Katalysatorbett 0,01 bis 15 Sekunden beträgt.

15. Verfahren nach einem oder mehreren der Ansprüche 1 bis 14, dadurch gekennzeichnet, daß der Druck innerhalb des Reaktors 0,1 bis 10 bar (0,01 bis 1,0 MPa) beträgt.

## Claims

1. A process for preparing pyrrolidone and N-alkyl pyrrolidones from saturated or unsaturated 1,4-dicarboxylic acids and/or their anhydrides, hydrogen and a primary amine or ammonia, characterised in that, in a suitable reactor, the dicarboxylic acid or its anhydride is allowed to react with hydrogen over a first catalyst bed and then the non-isolated intermediate is reacted directly with the primary amine or ammonia over a second, downstream catalyst bed.

2. A process according to Claim 1, characterised in that a copper-containing catalyst is used for the first catalyst bed.

3. A process according to Claim 1, characterised in that a silicon-containing catalyst or an aluminium phosphate catalyst is used for the second catalyst bed.

4. A process according to Claim 3, characterised in that the catalyst has a zeolitic structure.

5. A process according to one or more of Claims 1 to 4, characterised in that a copper chromite catalyst is used for the first catalyst bed and a cation-exchanged zeolite of the X or Y type is used for the second catalyst bed.

6. A process according to one or more of Claims 1 to 5, characterised in that the reaction takes place in the gas phase.

7. A process according to one or more of Claims 1 to 6, characterised in that diluents are present.

8. A process according to Claim 7, characterised in that the diluents are gases, water or aprotic solvents.

9. A process according to one or more of Claims 1 to 8, characterised in that the temperature in the reactor over the first reactor bed is 225 to 350°C.

10. A process according to one or more of Claims 1 to 9, characterised in that the temperature in the reactor over the second catalyst bed is 225 to 400°C.

11. A process according to one or more of Claims 1 to 10, characterised in that the temperature over the first catalyst bed is 250 to 300°C and over the second catalyst bed is 260 to 340°C.

12. A process according to one or more of Claims 1 to 11, characterised in that the molar ratio of the 1,4-dicarboxylic acid or its anhydride to hydrogen is 1 to 20 to 1 to 250.

13. A process according to one or more of Claims 1 to 12, characterised in that the molar ratio of hydrogen to primary amine or ammonia is 15 to 1 to 250 to 1.

14. A process according to one or more of Claims 1 to 13, characterised in that the residence time of the reaction partners over the particular catalyst bed is 0.01 to 15 seconds.

15. A process according to one or more of Claims 1 to 14, characterised in that the pressure inside the reactor is 0.1 to 10 bar (0.01 to 1.0 MPa).

## Revendications

1. Procédé pour la préparation de pyrrolidone et de N-alkylpyrrolidones à partir d'acides 1,4-dicarboxyliques saturés ou insaturés et/ou de leurs anhydrides, d'hydrogène et d'une amine primaire ou d'ammoniaque, caractérisé en ce que l'on fait réagir, dans un réacteur approprié sur un premier lit de catalyseur, l'acide dicarboxylique ou son anhydride avec de l'hydrogène, et en ce que l'on fait réagir ensuite directement le produit intermédiaire sans l'avoir isolé, sur un deuxième lit de catalyseur en aval du premier, avec l'amine primaire ou avec de l'ammoniaque.

2. Procédé conforme à la revendication 1, caractérisé en ce que l'on utilise pour le premier lit de catalyseur un catalyseur contenant du cuivre.

3. Procédé conforme à la revendication 1, caractérisé en ce que l'on utilise pour le deuxième lit de catalyseur un catalyseur contenant du silicium ou un catalyseur de type aluminium-phosphate.

4. Procédé conforme à la revendication 3, caractérisé en ce que le catalyseur a une structure de zéolithe.

5. Procédé conforme à une ou plusieurs des revendications 1 à 4, caractérisé en ce que l'on utilise pour le premier lit de catalyseur un catalyseur de type cuivre-chromite et pour le deuxième lit de catalyseur une zéolithe de type X ou Y ayant subi un échange de cations.

6. Procédé conforme à une ou plusieurs des revendications 1 à 5, caractérisé en ce que la réaction est réalisée en phase gazeuse.

7. Procédé conforme à une ou plusieurs des revendications 1 à 6, caractérisé en ce que des diluants sont présents.

8. Procédé conforme à la revendication 7, caractérisé en ce que les diluants sont des gaz, de l'eau ou des solvants aprotiques.

9. Procédé conforme à une ou plusieurs des revendications 1 à 8, caractérisé en ce que la température à l'intérieur du réacteur au-dessus du premier lit de catalyseur est de 225 à 350 °C.

10. Procédé conforme à une ou plusieurs des revendications 1 à 9, caractérisé en ce que la température à l'intérieur du réacteur au dessus du deuxième lit de catalyseur est de 225 °C à 400 °C.

11. Procédé conforme à une ou plusieurs des revendications 1 à 10, caractérisé en ce que la température au dessus du premier lit de catalyseur est comprise entre 250 et 300 °C et au dessus du deuxième lit de catalyseur entre 260 et 340 °C.

12. Procédé conforme à une ou plusieurs des revendications 1 à 11, caractérisé en ce que le rapport molaire de l'acide 1,4-dicarboxylique ou de son anhydride à l'hydrogène est compris entre 1/20 et 1/250.

13. Procédé conforme à une ou plusieurs des revendications 1 à 12, caractérisé en ce que le rapport molaire de l'hydrogène à l'amine primaire ou à l'ammoniaque est compris entre 15/1 et 250/1.

14. Procédé conforme à une ou plusieurs des revendications 1 à 13, caractérisé en ce que le temps de séjour des réactifs au dessus de chaque lit de catalyseur est compris entre 0,01 et 15 secondes.

15. Procédé conforme à une ou plusieurs des revendications 1 à 14, caractérisé en ce que la pression à l'intérieur du réacteur est comprise entre 0,1 et 10 bars (0,01 à 1,0 MPa).
